# EUROPÄISCHE PATENTSCHRIFT

(11) **EP 0 625 512 B1**
(45) Veröffentlichungstag und Bekanntmachung des Hinweises auf die Patenterteilung: **27.08.1997**
(21) Anmeldenummer: 94107200.1
(22) Anmeldetag: 09.05.1994
(51) Int. Cl.: C07D 239/04, C08K 5/3462, C09K 15/30

(54) **N-Alkyl-N'-Poly(oxyalkyl)hexahydropyrimidines**
N-Alkyl-N'-poly(oxyalkyl)hexahydropyrimidine
N-Alkyl-N'-poly(oxyalkyl)hexahydropyrimidines

(30) Priorität: 15.05.1993 DE 4316374
(43) Veröffentlichungstag der Anmeldung: 23.11.1994
(73) Patentinhaber: HOECHST AKTIENGESELLSCHAFT, 65929 Frankfurt am Main (DE)
(72) Erfinder: Hoffmann, Herrmann, Dr., D-65779 Kelkheim (DE); Kremer, Gernot, Dr., D-65779 Kelkheim (DE)

(56) Entgegenhaltungen:
- EP-A- 0 070 536
- US-A- 3 787 416
- US-A- 4 281 126

## Beschreibung

Korrosionsprobleme treten bei allen Prozessen der Erdölgewinnung und Erdölverarbeitung auf, bei denen Eisen oder eisenenthaltene Metalle mit wäßrigen Systemen in Berührung kommen. Besonders gravierend sind die Probleme bei der Einwirkung von Salzwasser, Kohlensäure und Schwefelwasserstoff. Die Schutzwirkungen der bekannten Handelsprodukte - meist werden Amine oder quaternäre Ammoniumverbindungen eingesetzt - ist aber häufig unzureichend oder verschlechtert sich nach kurzer Zeit, da die Verbindungen durch mechanische Einflüsse oder Reaktionen mit aggressiven Medien von der zu schützenden Metalloberfläche wieder abgelöst werden. Allein durch häufiges Nachdosieren kann das Problem nur selten gelöst werden.

In US-A-3 787 416 werden N-Hydroxyalkyl-N'-alkylhexahydropyrimidine und N-Alkoxyalkyl-N'-alkylhexahydropyrimidine sowie Verfahren zu deren Herstellung und deren Verwendung beschrieben. Diese Hexahydropyrimidine enthalten bevorzugt am N-Atom einen C₁-C₁₀-Alkyl-oxy-C₁-C₁₀-alkylrest bzw. einen Hydroxy-C₁-C₁₀-alkylrest und am N'-Atom einen sekundären C₃-C₄₀-Alkylrest bzw. einen C₄-C₁₂-Cycloalkylrest. Die beschriebenen Verbindungen besitzen vielfältige Verwendungsmöglichkeiten. Unter anderem werden sie als Zusatzstoffe zur Konservierung von organischen Substanzen, als Vernetzerkatalysatoren bei der Urethanherstellung, als Zwischenprodukte bei der Arzneimittelherstellung und auch als Korrosionsinhibitoren in Erdöl verwendet.

Insbesondere dann, wenn sich die Zusammensetzung von frischgefördertem Rohöl laufend ändert, z. B. wenn zur Erhöhung der Feldausbeute mit Salzwasser geflutet wird, ist die Gefahr der Schädigung der Anlagen durch Korrosion sehr hoch, so daß die Forderung nach neuen besseren Korrosionsschutzmitteln mit Langzeitwirkung gestellt wird. Weiterhin besteht auch Bedarf an Korrosionsinhibitoren, die auch in hochkonzentrierten Salzlösungen gelöst oder zumindest kolloiddispers verteilt sind.

Es wurde nun gefunden, daß durch Verwendung von N-Alkyl-N'-poly(oxyalkyl)hexahydropyrimidinen eine hervorragende Korrosionsschutzwirkung für Wasser/Öl-Emulsionen, wie sie beim Erdöl vorliegen, erreicht wird, wobei insbesondere die Langzeitwirkung im Vergleich zur herkömmlichen Korrosionsinhibitoren verbessert wird.

Gegenstand der Erfindung sind N-Alkyl-N'-poly(oxyalkyl)hexahydropyrimidine der Formel I 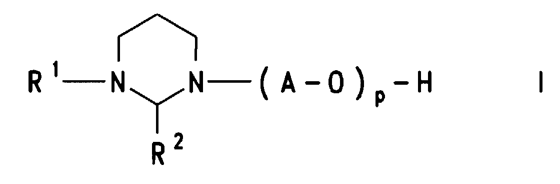 in der
R¹ C₁-C₃₀-Alkyl oder C₂-C₃₀-Alkenyl darstellt,
R² Wasserstoff oder C₁-C₃-Alkyl bedeutet,
A 1,2-Alkylengruppen mit 2 bis 10, bevorzugt 2 bis 5 C-Atomen bezeichnet und
p für eine Zahl von **3** bis 50 steht.

Als geradkettig oder verzweigte Alkyl- und Alkenylgruppen R¹ seien beispielsweise genannt: n-Hexyl, n-Heptyl, n-Oktyl, 2-Ethylhexyl, n- und iso-Nonyl, n- und iso-Decyl, n-Undecyl, n-Dodecyl, n-Tetradecyl, n-Hexadecyl, n-Octadecyl, Oleyl, Linolyl und Linolenyl.
Je nach Herkunft des bei der Synthese der Verbindung I eingesetzten primären Amins handelt es sich bei R¹ um Reste von natürlich vorkommenden Fettsäuren. Da die bei der Synthese der Verbindung I, bei denen R¹ Alkyl- oder Alkenylgruppen darstellt, in der Regel statistische Homologen- und auch Isomerengemische darstellen, ist es zweckmäßig, bei diesen Resten R¹ von einer durchschnittlichen Anzahl an C-Atomen zu sprechen.

Bevorzugt werden Verbindungen I, bei denen R¹ Alkyl oder Alkenylgruppen mit 9 bis 24 C-Atomen, insbesondere mit 10 bis 18 C-Atomen bedeuten. Besonders vorteilhaft sind solche Reste R¹, die auf die C₁₀-Fraktion, die C₁₃-Fraktion, den C₁₀/C₁₂-, den C₁₂/C₁₄-, den C₁₃/C₁₅- oder den C₁₆/C₁₈-Schnitt eines primären Amins zurückgeführt werden können.

Die 1,2-Alkylengruppen A bezeichnen insbesondere die Ethylen-, daneben auch die Propylen-, 1,2-Butylen- und 2,3-Butylengruppe. Dabei kann jede Gruppe A auch ein statistisches Gemisch aus mehreren der genannten 1,2-Alkylengruppen bezeichnen, wobei Gemische aus Ethylen und Propyleneinheiten bevorzugt sind.

Die Alkoxylierungsgrade p liegen zwischen **3** und 50, vorzugsweise 3 bis 35, insbesondere 5 bis 15. Die Werte für p stellen üblicherweise Durchschnittswerte dar.

Die erfindungsgemäßen Verbindungen der Formel I werden im allgemeinen durch Alkoxylierung der N-substituierten Hexahydropyrimidine der Formel IV erhalten. Als Alkoxylierungsreagenzien finden Epoxide, wie Ethylenoxid, Propylenoxid, Butylenoxid und 2,3-Epoxy-1-propanol Verwendung. Die Alkoxylierung der Verbindungen der Formel IV erfolgt nach an sich bekannten Verfahren. Geeignete Verfahren sind z.B. aus J. March, Advanced Organic Chemistry, 2. Auflage, S.357f (1977) bekannt.

Falls erforderlich können die Verbindungen der Formel IV nach dem folgenden Syntheseschema hergestellt werden.

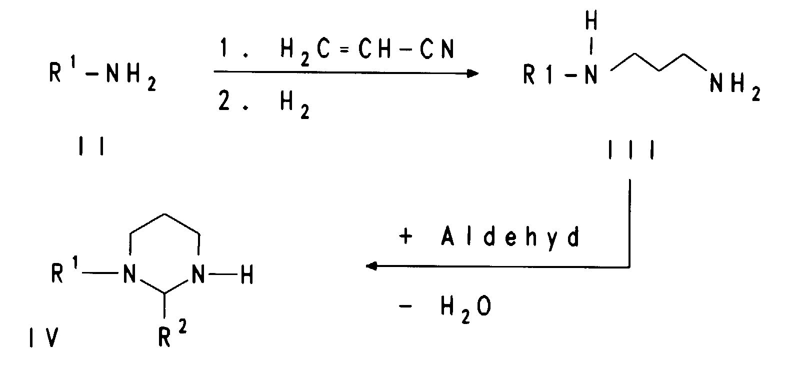

Ausgangsstoffe zur Herstellung der Verbindungen der Formel IV sind primäre Amine der Formel II. Soweit verfügbar werden handelsübliche primäre Amine eingesetzt. Leiten sich die Reste **R**^{**1**} von natürlich vorkommenden Fettsäuren ab, werden diese oder die entsprechenden Fette als Ausgangsstoffe zur Herstellung der primären Amine eingesetzt. Hierzu werden die Fette verseift, die erhaltenen Fettsäuren zum Nitril reduziert und aus dem Nitril durch Hydrierung das entsprechende primäre Fettamin gewonnen. Durch Umsetzung des primären Amins mit Acrylnitril und anschließender Hydrierung wird das N-substituierte Propylendiamin der Formel III erhalten, das in einer Kondensationsreaktion mit dem Aldehyd unter Wasserabspaltung zum N-substituierten Hexahydropyrimidin der Formel IV umgesetzt wird. Als Aldehyde werden Formaldehyd sowie Aldehyde mit einer C₁-C₃-Alkylgruppe eingesetzt. Bevorzugt werden Diamin der Formel III und Aldehyd in äquimolaren Mengen bei Temperaturen im Bereich von 80 bis 130°C umgesetzt. Zweckmäßigerweise wird das Diamin vorgelegt und der Aldehyd, bevorzugt in Form einer 35 Gew.-%igen Lösung, zugetropft. Üblicherweise wird während der Zugabe das vorhandene Wasser durch Destillation entfernt, wobei vorteilhafterweise die Temperatur erhöht wird, im allgemeinen auf bis zu 130°C und nach beendeter Zugabe gegebenenfalls leichtes Vakuum (20 bis 80 mbar) angelegt. Zur Vermeidung von Nebenprodukten, insbesondere Oxidationsprodukten, erfolgt die Herstellung der N-substituierten Hexahydropyrimidine der Formel IV unter Begasung mit Inertgas, bevorzugt Stickstoffbegasung. Die N-substituierten Hexahydropyrimidine der Formel IV werden im allgemeinen in guter Ausbeute und mit hohem Reinheitsgrad erhalten und können ohne weitere Reinigung zur nachfolgenden Alkoxylierung eingesetzt werden.

Eine bevorzugte Gruppe von Verbindungen der Formel I stellen Hexahydropyrimidine dar, die an dem N'-Atom eine Kette aus Ethylenoxid-Propylenoxid-Bausteinen tragen und durch die allgemeine Formel V 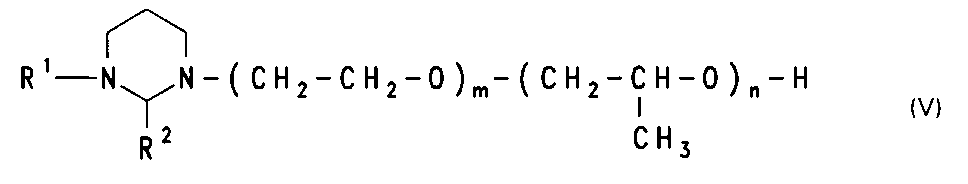 dargestellt werden, worin R¹ die vorstehend genannte Bedeutung besitzt und m und n gleich oder verschieden sind und Zahlen zwischen 0 und 50 bedeuten, wobei die Summe aus m und n eine Zahl zwischen **4** und 50 darstellt.

Die erfindungsgemäßen N-Alkyl-N'-poly(oxyalkyl)hexahydropyrimidine der Formel I eignen sich als Korrosionsinhibitoren, insbesondere in Erdölgewinnungs- und Verarbeitungsanlagen, die mit Salzwasser in Berührung kommen. Die Einsatzmengen dieser Verbindungen als Korrosionsinhibitoren betragen 1 bis 200, vorzugsweise 1 bis 50 mg pro Liter korrosive Flüssigkeit. Da die erfindungsgemäßen Verbindungen bei der Herstellung meist als höherviskose Flüssigkeiten anfallen erfolgt ihre Anwendung in der Praxis normalerweise als 20 - 50 gew.-%ige Lösung, z.B. in Wasser, Glykolen, Glykolethern, Alkoholen und anderen geeigneten Lösemitteln.
In diesen Lösungen können noch andere korrosionsinhibierende Wirkstoffe sowie Emulgiermittel, Antischaummittel und weitere übliche Zusätze vorhanden sein, die auf die Gebrauchseigenschaften des zur Anwendung gelangenden Produktes verbessernd wirken.
In der Regel wird der korrosionsinhibierende Effekt solcher Mischungen aber allein durch die erfindungsgemäßen Korrosionsinhibitorkomponenten bewirkt.

### Herstellungsbeispiele

Die Bestimmung des Oxethylierungsgrades erfolgt durch Titration des basischen Stickstoffs und durch Bestimmung des Trübungspunktes.

### 1. Herstellung von N-Talgfettalkylhexahydropyrimidin

341 g (1,0 Mol) Talgfettpropylendiamin werden vorgelegt und unter Stickstoffbegasung (ca. 5 l pro Stunde) auf 100°C erhitzt. Unter Rühren werden 85,7 g (1,0 Mol) 35 %ige Formaldehydlösung zugetropft, wobei gleichzeitig das entstehende Wasser abdestilliert wird. Nach beendeter Zugabe wird noch vorhandenes Wasser abdestilliert, bis die Innentemperatur ca. 130°C erreicht. Zur vollständigen Entfernung des vorhandenen Wassers wird bei dieser Temperatur ein leichtes Vakuum (ca. 20 mbar) über einen Zeitraum von ungefähr 2 Stunden angelegt. Nach Abkühlen auf Raumtemperatur werden 350 g einer öligen Flüssigkeit erhalten.
Aminzahl: 54,6 ml 0,1 N HCl/g (berechnet: 53,9 ml 0,1 N HCl/g)
Wasser (nach Karl Fischer): 0,15 Gew.-%

### 2. Alkoxylierung des N-Talgfettalkylhexahydropyrimidins

Die Alkoxylierung erfolgt in 2 Stufen. In der ersten Stufe wird ohne Katalysator 1 Mol Ethylenoxid angelagert, in der zweiten Stufe läßt man darüber hinaus bis zum gewünschten Umsetzungsgrad unter Einwirkung eines Katalysators (NaOH) mit Ethylenoxid bzw. Propylenoxid weiterreagieren.

### Erste Stufe:

1,0 Mol N-Alkylhexahydropyrimidin aus Beispiel 1 werden unter Stickstoffbegasung (1 bar) auf 160°C erhitzt. Über eine Druckbürette werden 48,4 g (1,1 Mol) Ethylenoxid während 1 Stunde zudosiert. Nach einer Nachrührzeit von 3 Stunden bei 160°C erhält man 390 g des entsprechenden N-Alkylhexahydropyrimidinoxethylats.

### Zweite Stufe:

1,0 Mol des N-Alkylhexahydropyrimidinoxethylats aus Stufe eins werden vorgelegt. Nach dem Eintragen von 1,5 g NaOH-Pulver wird Vakuum (20 mbar) angelegt und vorhandenes Wasser über einen Zeitraum von einer Stunde bei 90 bis 120°C entfernt. Anschließend wird bei 160°C die gewünschte Menge Ethylenoxid zudosiert. Gegebenenfalls erfolgt im Anschluß die Zugabe von Propylenoxid in analoger Weise. Tabelle 1 zeigt die hergestellten Beispiele 2 bis 10.

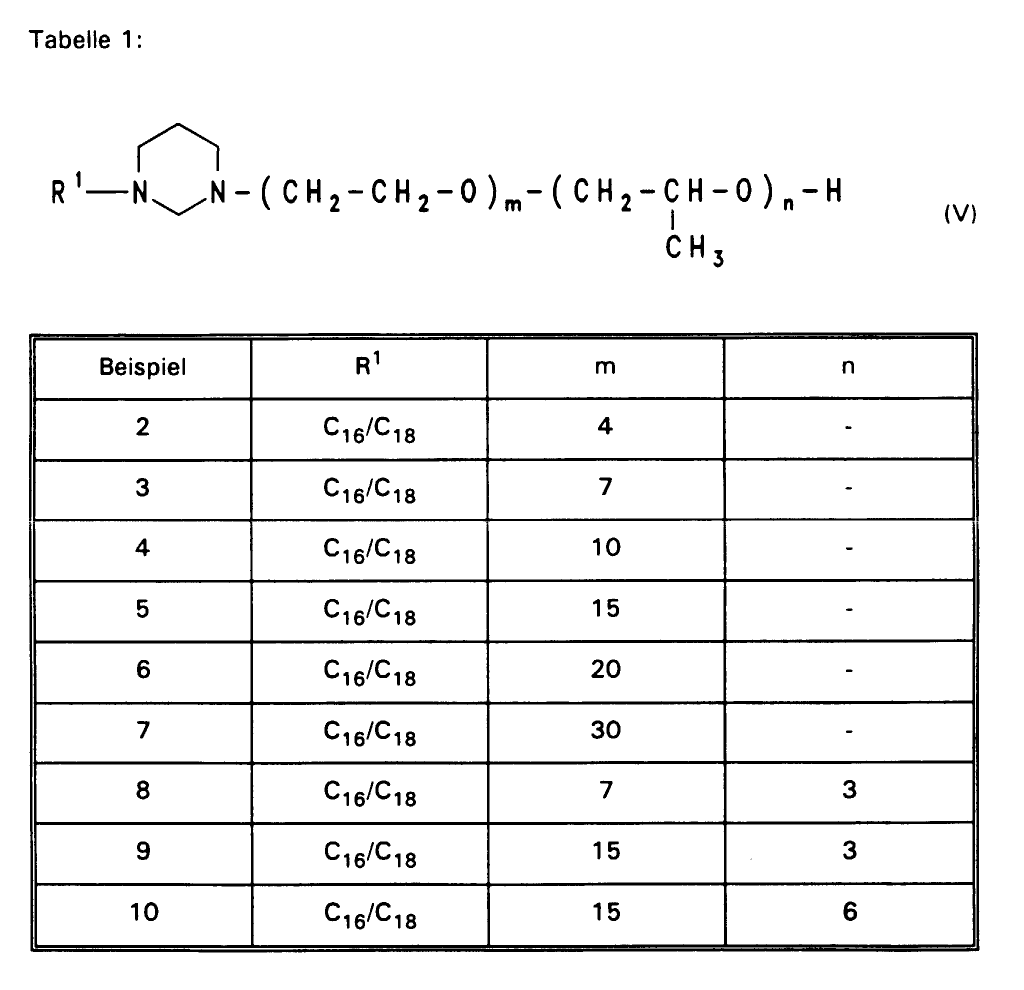

Die korrosionsinhibierende Wirkung wird über zwei Methoden nachgewiesen:

### 1) Wheel-Test

Der "Wheel-Test" ist eine gängige Methode für die Testung von Korrosionsinhibitoren für die Erdöl- und Erdgasförderung. Als Testcoupons werden Stahlbleche aus unlegiertem Stahl der Abmessungen 130 mm x 10 mm x 1 mm gewählt. Die Testcoupons werden leicht geschmirgelt, mit Aceton entfettet und vor und nach dem Korrosionsversuch gewogen. Als Testmedium dient Salzwasser mit 5 Gew.-% NaCl, das mit 5 % Kerosin überschichtet wird. Beide Phasen sind mit H₂S bzw. CO₂ gesättigt.
Dann werden 10, 20 bzw. 50 mg/l - bezogen auf das Gesamtvolumen der Flüssigkeit - an Inhibitor zugesetzt.
Die entfetteten und gewogenen Testcoupons werden anschließend in dieses Medium eingebracht und bei 70°C 24 Stunden einer mechanischen Bewegung (40 Upm mittels einer die Testgefäße drehenden Welle) unterzogen.
Die Testblechstreifen werden anschließend geschmirgelt, mit Aceton entfettet und nach Trocknung zur Bestimmung des Gewichtsverlustes gewogen. Die Korrosionsraten sind in mm/a (a =anno (Jahr)) angegeben. Zum Vergleich wird der Blindwert (Versuch ohne Inhibitorzusatz) ermittelt. Die Versuche mit Inhibitor werden mindestens einmal wiederholt und der Mittelwert der aus dem Gewichtsabtrag errechneten Korrosionsraten bestimmt. Als Blindwert wird der Streubereich von 10 Wiederholungen angegeben.
Die Ergebnisse des Wheel-Tests sind in Tab. 2 angegeben.

### 2) Autoklaventest

Zur Verwendung gelangt ein 4 l-Rührautoklav, an dessen Rührerwelle 8 Stahlcoupons à 10 cm² angeschraubt werden.
Folgende Bedingungen werden gewählt:
Medium: Wasser mit 5 % NaCl und mit Stickstoff sauerstofffrei gespült
Atmosphäre: 10 bar CO₂ bzw. 2 bar H₂S/4 bar CO₂
Temperatur: 120°C
Zeit: 24 Stunden,
Rührergeschwindigkeit: 800 Umdrehungen/min.
Meßprinzip: Gewichtsverlust.
Korrosionsraten: mm/a (Mittelwert von 8 Coupons)

Die Ergebnisse des Autoklaventests sind in Tabelle 3 angegeben.

**Tabelle 2:**

| | | | |
|---|---|---|---|
| Ergebnisse des Wheel-Tests | | | |
| Korrosionsraten: mm/a | | | |
| Atmosphäre: CO₂ (1 bar) | | | |
| Temperatur: 70°C | | | |
| Zeitdauer: 24 Stunden | | | |
| Medium: NaCl-Lsg. (5 gew.-%ig) : Kerosin (9:1), mit Stickstoff sauerstofffrei gespült. | | | |

| Beispiel | 2 mg/l | 4 mg/l | 20 mg/l |
|---|---|---|---|
| 2 | 0,30 | 0,25 | 0,17 |
| 3 | 0,25 | 0,18 | 0,16 |
| 4 | 0,21 | 0,17 | 0,16 |
| 5 | 0,23 | 0,19 | 0,16 |
| 6 | 0,33 | 0,22 | 0,18 |
| 7 | 0,35 | 0,18 | 0,20 |
| 8 | 0,39 | 0,33 | 0,22 |
| 9 | 0,30 | 0,30 | 0,19 |
| 10 | 0,29 | 0,23 | 0,19 |
| Cocosdimethylbenzylammoniumchlorid (Vergleich) | 0,35 | 0,30 | 0,30 |
| Blindwert | 0,70 - 0,85 | | |

**Tabelle 3**

| Ergebnisse des Autoklaventests | | |
|---|---|---|
| Beispiel | 10 bar CO₂/120°C | 2 bar H₂S/4 bar CO₂/120°C |
| 2 | 1,95 ± 0,20 mm/a | 0,35 ± 0,05 mm/a |
| 3 | 1,21 ± 0,15 mm/a | 0,37 ± 0,03 mm/a |
| 4 | 0,95 ± 0,15 mm/a | 0,20 ± 0,02 mm/a |
| 5 | 1,35 ± 0,23 mm/a | 0,54 ± 0,03 mm/a |
| 6 | 1,31 ± 0,06 mm/a | 0,34 ± 0,06 mm/a |
| 7 | 1,53 ± 0,09 mm/a | 0,46 ± 0,04 mm/a |
| 8 | - | - |
| 9 | 1,70 ± 0,33 mm/a | - |
| 10 | - | - |
| Amidamin aus 2 Mol Naphthensäure + 1 Mol Diethylentriamin (Vergleich) | 3,7 ± 0,3 mm/a | 0,51 ± 0,06 mm/a |
| Cocosdimethylbenzylammoniumchlorid (Vergleich) | 2,3 ± 0,17 mm/a | 0,80 ± 0,05 mm/a |
| Blindwert | 10,7 ± 1,2 mm/a | 4,8 ± 0,5 mm/a |

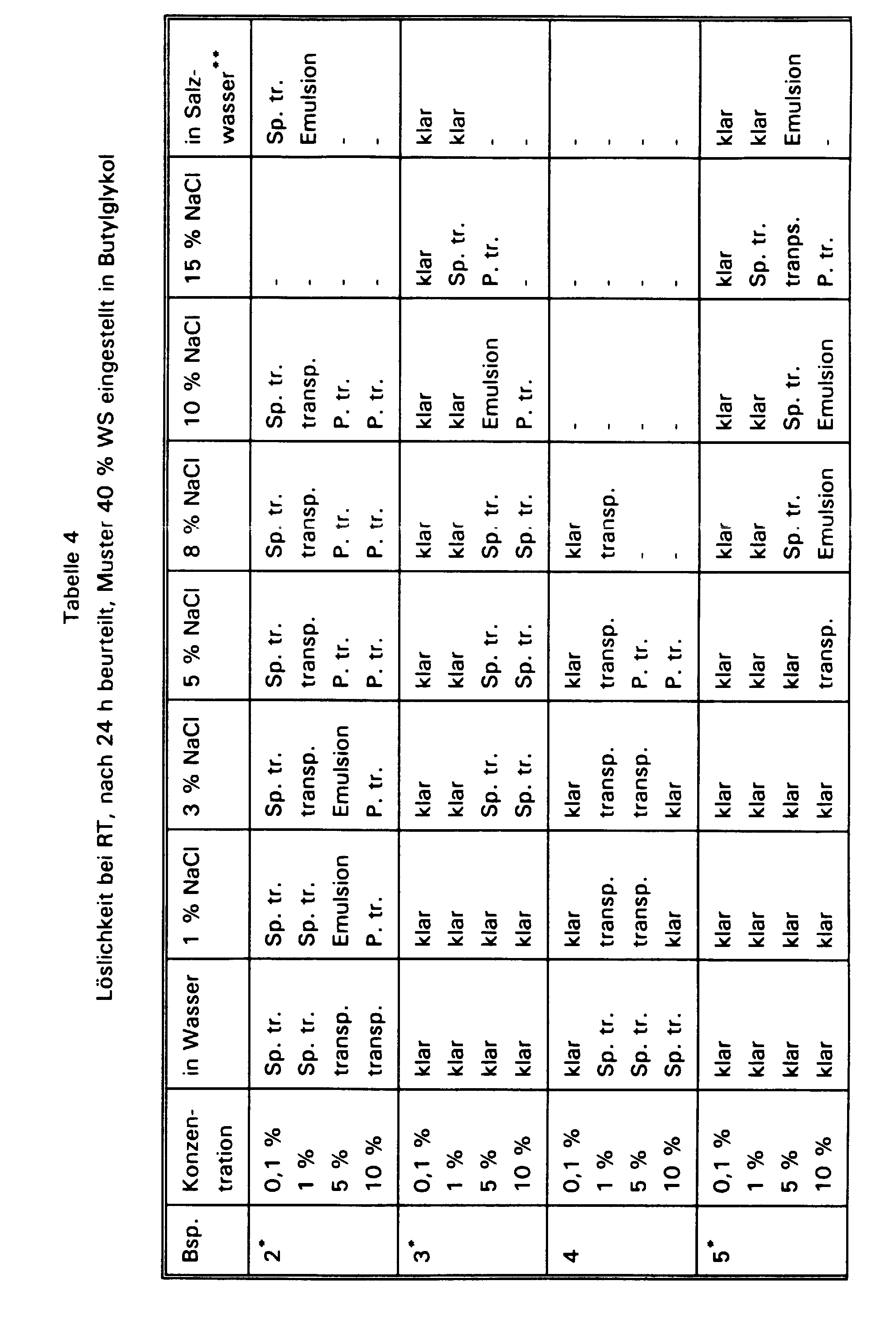

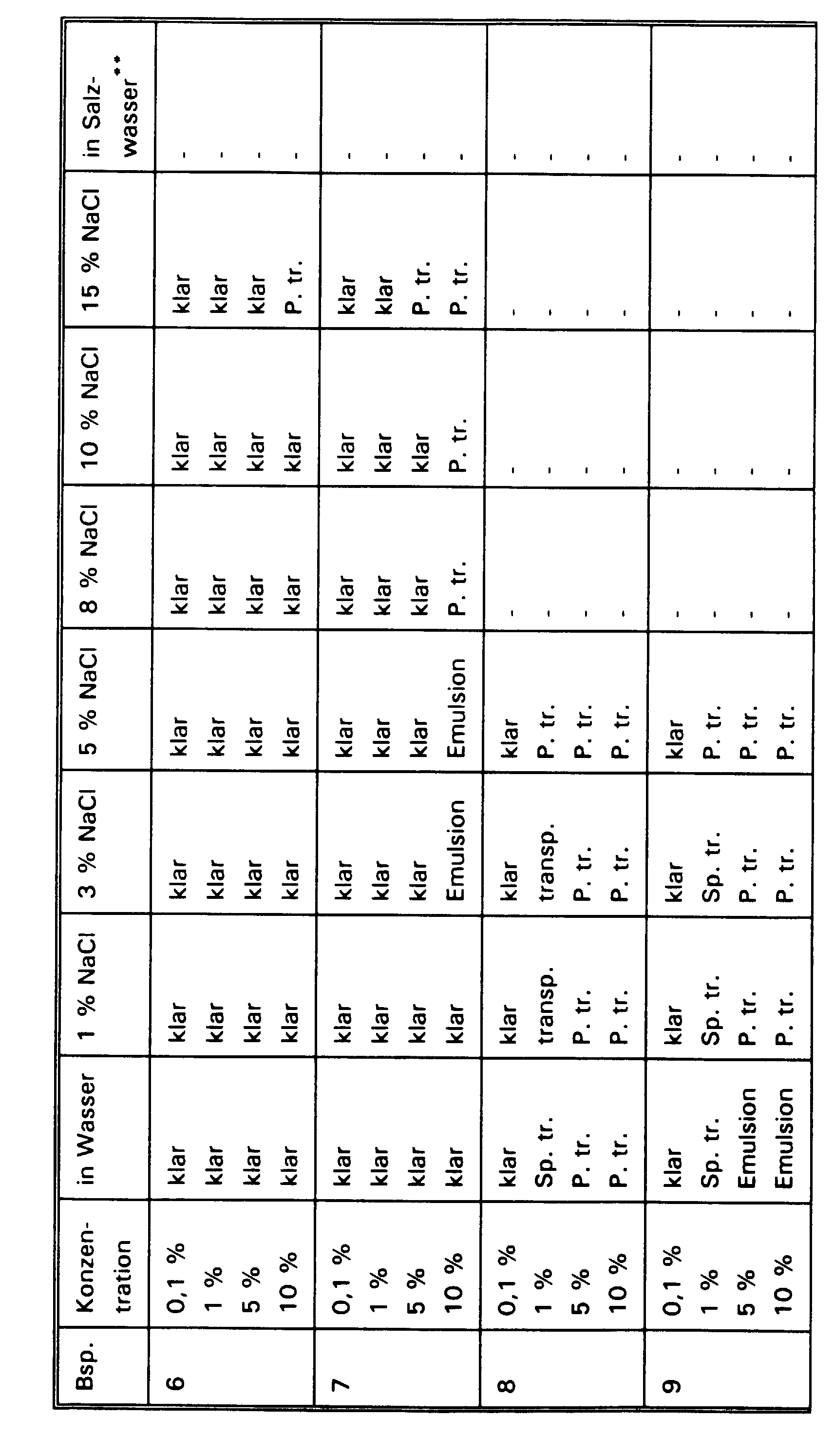

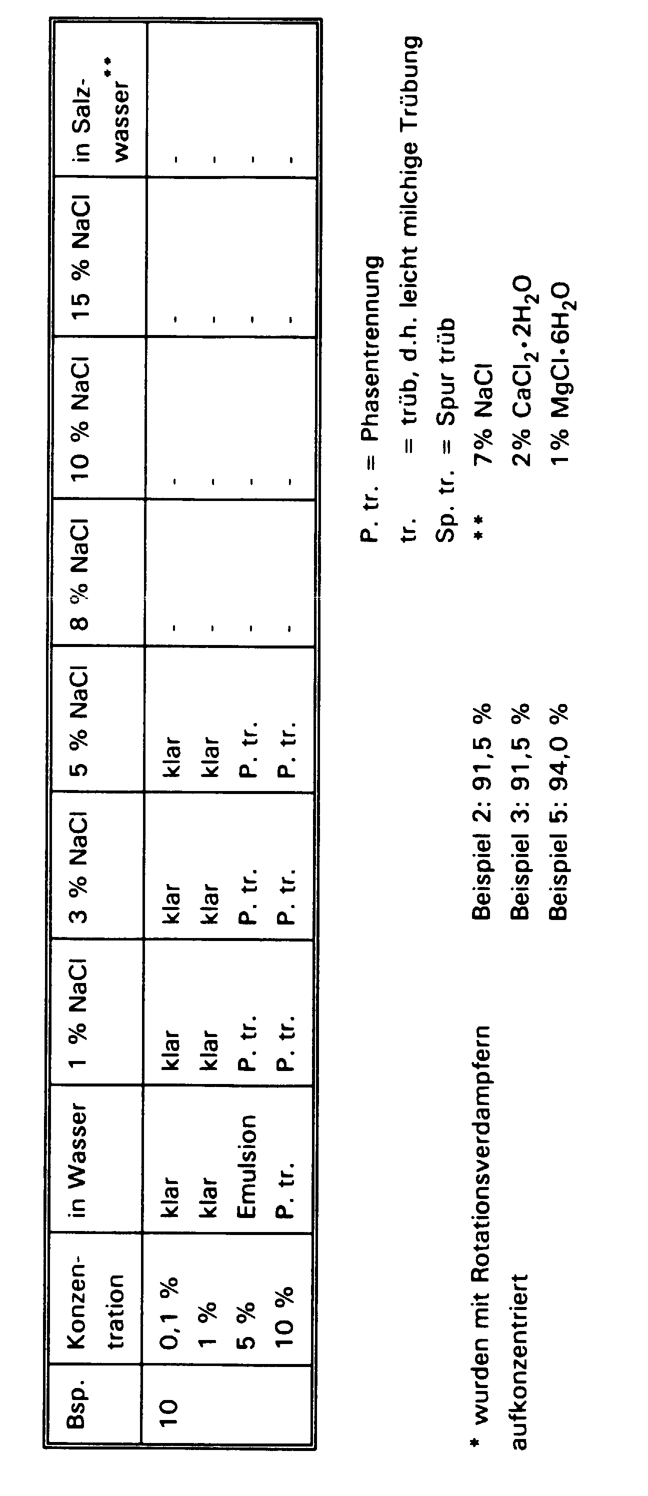

## Patentansprüche

1. N-Alkyl-N'-poly(oxyalkyl)hexahydropyrimidine der Formel I 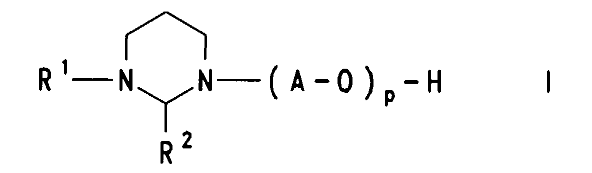 worin
R¹ C₁-C₃₀-Alkyl oder C₂-C₃₀-Alkenyl darstellt,
R² Wasserstoff oder C₁-C₃-Alkyl bedeutet,
A 1,2-Alkylengruppen mit 2 bis 10, bevorzugt 2 bis 5-C-Atomen bezeichnet und
p für eine Zahl von **3** bis 50 steht.

2. Verbindungen der Formel I nach Anspruch 1, dadurch gekennzeichnet, daß
p für eine Zahl von 3 bis 35 steht.

3. Verbindungen der Formel I nach Anspruch 1 oder 2, dadurch gekennzeichnet, daß
R¹ C₁₀-C₁₈-Alkyl oder C₉-C₂₄-Alkenyl darstellt.

4. Verbindungen der Formel I nach Anspruch 1, dadurch gekennzeichnet, daß A eine Ethylengruppe und/oder eine Propylengruppe bezeichnet und diese Verbindungen die Formel V besitzen 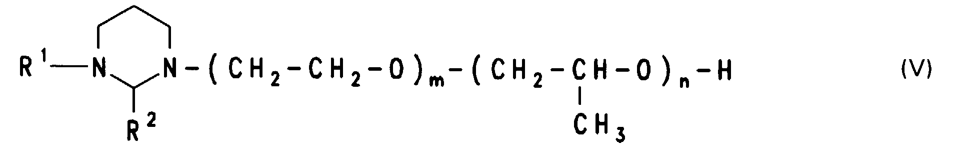 worin
m und n gleich oder verschieden sind und Zahlen zwischen 0 und 50 bedeuten,
wobei die Summe aus m und n eine Zahl zwischen **4** bis 50 darstellt.

5. Verfahren zur Herstellung der Verbindungen der Formel I nach Anspruch 1 durch Alkoxylierung von N-substituierten Hexahydropyrimidinen der Formel IV 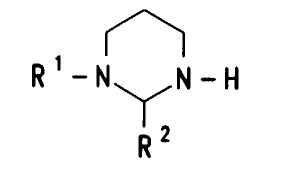 in der
R¹ C₁-C₃₀-Alkyl oder C₂-C₃₀-Alkenyl darstellt und
R² Wasserstoff oder C₁-C₃-Alkyl bedeutet.

6. Verwendung von Verbindungen nach Anspruch 1 als Korrosionsinhibitoren, insbesondere in Erdölgewinnungs- und Erdölverarbeitungsanlagen.

## Claims

1. An N-alkyl-N'-poly(oxyalkyl)hexahydropyrimidine of the formula I 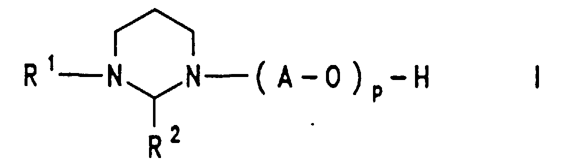 in which
R¹ is C₁-C₃₀-alkyl or C₂-C₃₀-alkenyl,
R² is hydrogen or C₁-C₃-alkyl,
A is a 1,2-alkylene group having from 2 to 10, preferably from 2 to 5, carbon atoms and
p is a number from 3 to 50.

2. A compound of the formula I as claimed in claim 1, wherein
p is a number from 3 to 35.

3. A compound of the formula I as claimed in claim 1 or 2, wherein
R¹ is C₁₀-C₁₈-alkyl or C₉-C₂₄-alkenyl.

4. A compound of the formula I as claimed in claim 1, wherein A is an ethylene group and/or a propylene group and this compound has the formula V 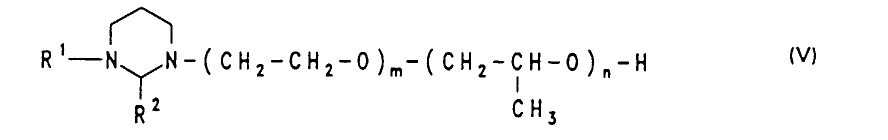 in which
m and n are identical or different and are numbers between 0 and 50, the sum of m and n being a number between 4 and 50.

5. A process for preparing a compound of the formula I as claimed in claim 1 by alkoxylation of an N-substituted hexahydropyrimidine of the formula IV 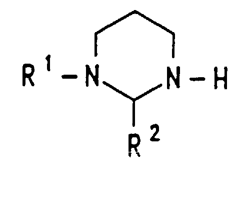 in which
R¹ is C₁-C₃₀-alkyl or C₂-C₃₀-alkenyl and
R² is hydrogen or C₁-C₃-alkyl.

6. Use of compounds as claimed in claim 1 as corrosion inhibitors, in particular in petroleum extraction and petroleum processing plants.

## Revendications

1. N-alkyl-N'-poly(oxyalkyl)hexahydropyrimidine de formule I 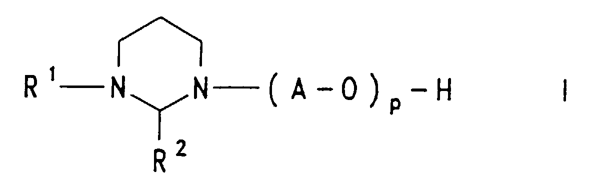 dans laquelle
R¹ représente alkyle en C₁-C₃₀ ou alcényle en C₂-C₃₀,
R² représente l'hydrogène ou alkyle en C₁-C₃,
A représente des groupes 1,2-alkylène avec 2 à 10, de préférence avec 2 à 5 atomes de carbone
et
p va de 3 à 50.

2. Composés de formule I selon la revendication 1, caractérisés en ce que
p va de 3 à 50.

3. Composés de formule I selon la revendication 1 ou 2, caractérisés en ce que
R¹ représente alkyle en C₁₀-C₁₈ ou alcényle en C₉-C₃₄.

4. Composés de formule I selon la revendication 1, caractérisés en ce que A représente un groupe éthylène ou un groupe propylène et ces composés de formule V répondent à la formule 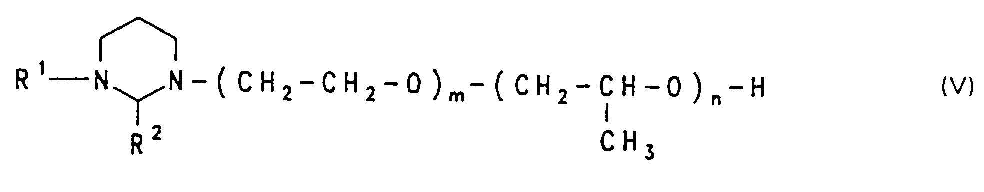 dans laquelle
m et n sont identiques ou différents et représentent des nombres compris entre 0 et 50, la somme de m et de n représente un nombre compris entre 4 et 50.

5. Procédé pour la préparation des composés de formule I selon la revendication 1, par alcoxylation des hexahydropyrimidines N-substitués de formule IV 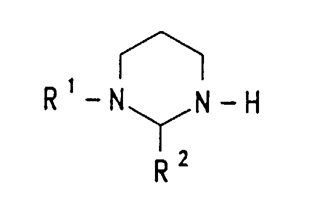 dans laquelle
R¹ représente alkyle en C₁-C₃₀ ou alcényle en C₂-C₃₀ et
R² représente l'hydrogène ou alkyle en C₁-C₃.

6. Utilisation des composés selon la revendication 1 comme inhibiteurs de corrosion, notamment dans les installations d'extraction et de traitement de pétrole.
